# EUROPEAN PATENT SPECIFICATION

(11) **EP 2 699 259 B1**
(45) Date of publication and mention of the grant of the patent: **27.07.2016**
(21) Application number: 12714730.4
(22) Date of filing: 20.04.2012
(51) Int. Cl.: C07K 16/30, C07K 16/28, G01N 33/574

(54) **BCMA-BASED STRATIFICATION AND THERAPY FOR MULTIPLE MYELOMA PATIENTS**
BCMA-BASIERTE STRATIFIZIERUNG UND THERAPIE FÜR PATIENTEN MIT MULTIPLEM MYELOM
STRATIFICATION BASÉE SUR BCMA ET THÉRAPIE POUR LES PATIENTS ATTEINTS DE MYÉLOME MULTIPLE

(30) Priority: 21.04.2011 EP 11163558
(43) Date of publication of application: 26.02.2014
(73) Proprietor: Boehringer Ingelheim International GmbH, 55216 Ingelheim am Rhein (DE)
(72) Inventor: BORGES, Eric, 55216 Ingelheim Am Rhein (DE); HEBEIS, Jasmin Barbara, 55216 Ingelheim Am Rhein (DE)
(74) Representative: Simon, Elke Anna Maria
(86) International application number: PCT/EP2012/057248
(87) International publication number: WO 2012/143498

(56) References cited:
- RYAN MAUREEN C ET AL: "Antibody targeting of B-cell maturation antigen on malignant plasma cells", MOLECULAR CANCER THERAPEUTICS, AMERICAN ASSOCIATION OF CANCER RESEARCH, US, vol. 6, no. 11, 1 November 2007 (2007-11-01), pages 3009-3018, XP002581270, ISSN: 1535-7163
- P MOREAU ET AL: "Rituximab in CD20 positive multiple myeloma", LEUKEMIA, 1 February 2007 (2007-02-01), XP055004458, ISSN: 0887-6924, DOI: 10.1038/sj.leu.2404558
- A. J. NOVAK: "Expression of BCMA, TACI, and BAFF-R in multiple myeloma: a mechanism for growth and survival", BLOOD, vol. 103, no. 2, 15 January 2004 (2004-01-15), pages 689-694, XP055004448, ISSN: 0006-4971, DOI: 10.1182/blood-2003-06-2043
- Dispenzieri ET AL: "Treatment of Newly Diagnosed Multiple Myeloma Based on Mayo Stratification of Myeloma and Risk-Adapted Therapy (mSMART): Consensus Statement", Mayo Clin Proc 82(3), 1 March 2007 (2007-03-01), pages 323-341, XP055004528, Retrieved from the Internet: URL:http://mmfighters.home.comcast.net/~mm fighters/TREATMENTNEWLYDIAGNOSEDMULTIPLEMY ELOMA.pdf [retrieved on 2011-08-10]
- YU-TZU TAI ET AL: 'Antibody-Based Therapies in Multiple Myeloma' BONE MARROW RESEARCH vol. 27, no. 15S, 26 March 2011, pages 1 - 14, XP055138461 DOI: 10.1182/blood-2009-02-206532 ISSN: 2090-2999

### SEQUENCE LISTING

<110> Boehringer Ingelheim International GmbH
<120> BCMA-based stratification and therapy for multiple myeloma patients
<130> 12-0333-pct
<160> 3
<170> PatentIn version 3.5
<210> 1
   <211> 184
   <212> PRT
   <213> Homo sapiens
<400> 1
<210> 2
   <211> 24
   <212> DNA
   <213> artificial
<220>
   <223> synthetic oligonucleotide derived from the human BCMA gene
<400> 2
   ttacttgtcc ttccaggctg ttct 24
<210> 3
   <211> 25
   <212> DNA
   <213> artificial
<220>
   <223> synthetic oligonucleotide derived from the human BCMA gene
<400> 3
   catagaaacc aaggaagttt ctacc 25

## Description

The present invention relates to methods for the stratification of a multiple myeloma (MM) patient comprising determining whether or not B-cells, preferably malignant B-cells of said patient express BCMA protein on their surface. Also, methods for selecting an antibody-based multiple myeloma (MM) therapy is based on whether or not BCMA is expressed on the cell surface of B-cells, preferably malignant B-cells of a patient. Furthermore, antibody-based therapies for patients who have BCMA positive malignant B-cells are provided.

### BACKGROUND OF THE INVENTION

Multiple myeloma (MM), an incurable malignancy of the plasma B-cells, accounts for an estimated 14% of all newly diagnosed hematologic malignancies (Colson et al., (2004) Clin J Oncol Nurs 8 (5): 473-480). MM is a heterogenous disease and caused by mostly b y chromosome translocations *inter alia* t(11;14), t(4;14), t(8;14), del(13), del(17) (Drach et al., (1998) Blood 92(3):802-809;

Gertz et al., (2005) Blood 106(8):2837-2840; Facon et al., (2001) Blood 97(6):1566-1571). MM- affected patients may experience a variety of disease-related symptoms due to bony destruction, bone marrow infiltration, renal failure, immunodeficiency, and the psychosocial burden of a cancer diagnosis. Exciting new therapies such as chemotherapy and stem cell transplantation approaches are becoming available and have improved survival rates but often bring unwanted side effects, and thus MM remains still incurable (Lee et al., (2004) J Natl Compr Canc Netw 8 (4): 379-383).

MM is caused by malignancy of the plasma B-cells. In order to finally identify said malignant plasma B-cells, it was found that several proteins reside on the surface of plasma B-cells (Harada et al., (1993) Blood 81(10): 2658-2663; Robillard N et al., Blood (2003) 102(3): 1070-1071). For instance, CD38 was found to express strongly on the surface of myeloma cells (malignant plasma B-cells), i.e. CD38⁺⁺ and therefore to distinguishing plasma B-cells from other hematopoietic cells in the bone marrow. In addition, Harada et al also found that normal plasma B-cells were VLA-4⁺ VLAS-5⁺ MPC-1⁺ CD44⁺ CD19⁺ CD56⁻ in the bone marrows from 7 healthy donors, on the other hand, mature myeloma cells (12 of 20 cases) were VLA-4⁺ VLAS-5⁺ MPC-1⁺ CD19⁻ CD56⁺ and no myeloma cells were CD19⁺Cd56⁻. In addition, it was reported that expression of CD20 is associated with small mature plasma B-cell morphology and MM patients with t(11;14).

BLyS (B Lymphocyte stimulator), also referred to as BAFF, TALL-1, or THANK, is a tumor necrosis factors (TNF) superfamily member. It is a type II transmembrane protein and plays a significant role in maintaining the development and homeostasis of normal B-cell and promotes the survival of malignant B cells. Three receptors for BLyS were identified, i.e. BCMA (B cell maturation antigen), BAFF receptor (BAFF-R), and TACI (transmembrane activator and calcium modulator cyclophilin ligand interactor). These three receptors are type I single membrane receptors and belong to the TNF receptors family. BCMA and BAFF-R are predominantly expressed on B lymphocytes, while TACI can be found on B cells and activates T cells. In addition, BCMA and TACI are capable of binding to APRIL (a proliferation-inducing ligand) which is the closest structural homologue of BLyS.
Novak et al found that three receptors for BLyS (BAFF) are expressed on the cell surface of B-cells of MM patients (Novak et al., (2004) Blood 103(2):689-694).

One of receptors for BLyS, BCMA, is known to be preferentially expressed in mature B cells (Gras et al., (1995) Int Immunol 7:1093-1106; Thompson et al., (2000) J Exp Med 192:129-135). Further, it was found that the expression of *BCMA* as BCMA mRNA was up-regulated during the late stages of normal B-cell differentiation and was highly expressed in MM cells (Tarte et al., (2002) Blood 100:1113-1122; Tarte et al., (2003) Blood 102:592-600; Claudio et al., (2002) Blood 100:2175-2186). Moreover, Bellucci et al confirmed that expression of *BCMA,* i.e. BCMA mRNA showed selective expression in late stages of B-cell maturation (Belluci et al., (2005) Blood 105:3945-3950). However, Li et al found that expression of *BCMA,* i.e. BCMA mRNA was not always detectable in MM cells from patients (Li et al., (2010) Med Oncol 27:439-445). To this end, even though it has been reported that BCMA expression is limited only in the plasma cells and germinal center B cells, a BCMA+ population was also observed in naïve and memory B cells from peripheral blood (US2009/0325196). Average of BCMA+ B cells are 24% in naïve B cells and 20.8% in memory B cells, which is lower than that of plasmoblasts (37.9%). However, it seems that BCMA is mainly expressed in plasmablasts.

It has been reported that t(4;16)(q26;p13.1) chromosome translocation was found in tumor cells from a patient with a T cell lymphoma, resulting in the fusion of BCMA gene and interleukin 2 gene, and t(16;18)(p13;q21.3) chromosome translocation was found in a male patient with follicular lymphoma, resulting in the fusion of BCMA gene, HLA-D gene and several hematopoietic neoplasm-related genes such as MHC2TA with BCL2 gene (Laabi et al., (1992) J EMBO 11(11):3897-3904;Mahmoodi et al., (2004) Can Genet Cytogenet 154(2):160-162). Although these chromosomal translocations were not reported to cause MM, one of translocation is related to follicular lymphoma which is defined as a lymphoma of follicle center B cells.

Furthermore, Novak et al found that MM cell lines and freshly isolated MM cells express BCMA protein and TACI protein on their cell surfaces and have variable expression of BAFF-R protein on their cell surface (Novak et al., (2004) Blood 103(2):689-694).

It was also reported that BCMA protein is expressed on the cell surface of MM cells/cell lines although BCMA protein is originally reported as an integral membrane protein in the Golgi apparatus of human mature B lymphocytes, i.e. as an intracellular protein (Gras et al., (1995) International Immunol 7(7):1093-1105), which shows that BCMA seems to have an important role during B-cell development and homeostasis. The finding of Gras et al. might be associated with the fact that the BCMA protein that was described in Gras et al. is, because of a chromosomal translocation, a fusion protein between BCMA and IL-2. However, BCMA is meanwhile established to be a B-cell marker that is essential for B-cell development and homeostasis (Schliemann et al., (2001) Science 293 (5537):2111-2114) due to its presumably essential interaction with BAFF and APRIL.

Thus far, the two most promising treatment options for patients with multiple myeloma are tandem high-dose chemotherapy followed by autologous stem cell infusion or allogeneic hematopoietic stem cell transplantation after myelo-ablative therapy or reduced-intensity conditioning. However, cure is rarely achieved due to persistence of minimal residual disease. Thus, there is an urgent need for innovative treatment modalities to stabilize or even eradicate residual tumor cells.
With the discovery and molecular characterization of cancer target antigens, more focused approaches may become available to steer the patient's immune system to recognize and eliminate residual cancer cells after conventional ablative therapies. Antibody-based therapies are thus an attractive option for the treatment of MM. Since MM cells have been B-cells prior to their "conversion", commonly known and specific B-cell surface proteins are used as targets for antibodies such as CD20. Though normal B-cells will also be targeted by an anti-CD20 antibody, an anti-CD20 antibody therapy is applied to patients, since it was observed that normal B-cells will recover, while it is assumed (and has been observed) that malignant B-cells will not recover. Thus far, those of skill in the art, start from the assumption that once malignant B-cells express the target of interest on mRNA level such as BCMA, PIM2, MUM1/IRF4 or XBP1 (Claudio et al. (2002), Blood 100:2175-2186), such cells can be subject to an antibody-based therapy.

However, thus far it has not yet been reported as to whether or not there is a correlation of BCMA mRNA and BCMA protein in MM patients as well as in MM cell lines to evaluate a possible therapy for MM using, in particular, an anti-BCMA antibody. Rather, in accordance with the commonly accepted concept that DNA makes RNA makes protein, it was rightly assumed that once BCMA mRNA is detectable in MM cells, BCMA protein is present on the cell surface of said cells to act as receptor. This would be in line with various reports that assign BCMA an important role in the development of B-cell immunity and B-cell homeostasis (Mackay and Browning (2002) Nat Rev Immunol. (2):465-475) as well as in proliferation of MM cells (Novak et al., cited above; Bellucci et al., (2005) Blood 105:3945-3950).

Several recent studies have also documented the expression of BCMA in malignant as well as normal plasma cells and have shown that BAFF signaling through BCMA results in myeloma cell proliferation (Shu et al., (2000) Proc Natl Acad Sci USA 97:9156-9161; O'Connor et al., (2004) J Exp Med. 199:91-98; Novak et al., cited above; Avery et al., (2003) J Clin Invest. 112:286-297). Taken together, these studies suggested that BCMA is commonly expressed in myeloma and signaling through BCMA may also contribute to the expansion of myeloma cells in vivo (Bellucci et al., cited above).

Accordingly, based on the commonly accepted concept DNA makes RNA makes protein and given the fact that BCMA is assigned an important role in MM proliferation, thus implying its presence on the cell surface of MM B-cells, multiple myeloma treatment would automatically have been initiated (Ryan et al., (2007) Mol Cancer Ther 6: 3009-3018).
Consequently, no one has thus far questioned as to whether or not BCMA is indeed present on the cell surface, if its mRNA is detectable, and therefore an antibody-based therapy would have been initiated, though it would not necessarily be beneficial for patients suffering from multiple myeloma, if BCMA is not present on the cell surface.

The present inventors, despite the knowledge that BCMA seems to be important both in normal B-cell development and malignant B-cell proliferation (in multiple myeloma), have nevertheless asked themselves the question as to whether or not and despite the important role of BCMA, also in MM, the dogma DNA makes RNA makes protein which is then presumably present on the cell surface would hold true, if the dogma is only based on BCMA mRNA data. Put it differently, it was not recognized in the art that BCMA, despite detectable mRNA, might not be present on the cell surface of MM cells, thus being not available as target for an antibody-based therapy (Ryan et al., cited above). Accordingly, no one in the art has thought of personalizing an anti-BCMA antibody based therapy for treating or ameliorating MM, since it was thought DNA makes RNA makes protein which is present on the surface of MM cells, bearing in mind the important role of BCMA in malignant B-cell development and proliferation. Therefore, a need exists for personalizing an anti-BCMA antibody therapy.

Hence, the technical problem underlying the present invention is to comply with the needs described above.

### DESCRIPTION OF THE FIGURES

**Figure 1****:** Binding of anti-BCMA antibodies to OPM-2 cells by FACS in OPM-2 cells
**Figure 2****:** BCMA Protein (A) and BCMA mRNA (B) expression levels of 10 different MM cell lines.
**Figure 3****:** Expression analysis of primary MM cells - exemplary results of positive and negative BCMA expressing patient samples

### SUMMARY OF THE INVENTION

The present invention addresses these needs by providing a therapy using anti-BCMA antibodies, said therapy is based on the present inventors' recognition that the assumption that if BCMA mRNA is detectable BCMA protein is, so to say, automatically present on the surface of MM cells not necessarily correct for each and every patient. Indeed, the present inventors have found that it is of utmost importance to screen MM patient for the presence or absence, respectively, of the expression of BCMA on the protein level, i.e., for its presence or absence, respectively, on the cell surface of MM cells.

Accordingly, the present invention provides a method for the stratification of a multiple myeloma (MM) patient disposed to respond favorably to an anti-BCMA antibody therapy, comprising determining whether B-cells of said patient, preferably malignant B-cells (MM cells or MM B-cells) expresses BCMA protein on the surface of said B-cells, wherein said patient is disposed to respond favorably to said anti-BCMA antibody therapy, if said B-cells express BCMA protein on their surface.

Furthermore, the specification provides a method for diagnosing a BCMA negative multiple myeloma (MM) patient, comprising determining whether B-cells, preferably malignant B-cells, of said patient express BCMA protein on their surface, wherein said patient suffers from BCMA negative MM, if no BCMA protein is detectable on the surface of said B-cells.

Also, the present specification provides a method for selecting an antibody-based multiple myeloma (MM) therapy, comprising determining whether B-cells of a patient, preferably malignant B-cells, express BCMA protein on the surface of said B-cells, wherein, if said B-cells are BCMA positive, the patient may be subject to an anti-CD20 antibody therapy and/or an anti-CD38 antibody therapy and/or an anti-BCMA antibody therapy and/or an anti-CS1 antibody therapy, or, if said B-cells are BCMA-negative, the patient is subject to an anti-CD20 antibody therapy and/or an anti-CD38 antibody therapy and/or an anti-CS1 antibody therapy.

Further aspects relate to an anti-BCMA antibody therapy for use in the treatment or amelioration of a multiple myeloma (MM) patient whose B-cells are disposed to be BCMA positive, an anti-BCMA antibody for use in the treatment or amelioration of a multiple myeloma (MM) patient diagnosed in accordance with the method(s) of the present invention, and an anti-CD20 antibody and/or an anti-CD38 antibody and/or an anti-CS1 antibody therapy for use in the treatment or amelioration of a multiple myeloma (MM) patient whose B-cells are BCMA negative.

It must be noted that as used herein, the singular forms "a", "an", and "the", include plural references unless the context clearly indicates otherwise. Thus, for example, reference to "an expression cassette" includes one or more of the expression cassettes disclosed herein and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein.

Throughout this specification unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or sometimes when used herein with the term "having".
When used herein "consisting of" excludes any element, step, or ingredient not specified in the claim element. When used herein, "consisting essentially of" does not exclude materials or steps that do not materially affect the basic and novel characteristics of the claim. In each instance herein any of the terms "comprising", "consisting essentially of" and "consisting of" may be replaced with either of the other two terms.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or" as used herein.

Several documents are cited throughout the text of this specification.

An object is to the provide a method for stratification of MM patients based on BMCA expression on the surface of the patients' B-cells, preferably malignant B-cells and new therapy for MM patients using an anti-BCMA antibody, preferably combined with other antibodies such as anti-CD38, anti-CD20 and/or anti-CS1 antibodies based on the correlation of expression of *BCMA* gene, i.e. BCMA mRNA and BCMA protein on the surface of B-cells, preferably malignant B-cells of MM patients.
Given the fact that BCMA is not or essentially not expressed on each and every healthy B-cell of a patient, but on malignant B-cell of a patient, if said patient is diagnosed by the methods of the present invention as BCMA positive, an anti-BCMA antibody therapy may be superior in comparison to, for example, an anti-CD20 antibody therapy, since CD20 is believed to be expressed on each and every B-cell. Accordingly, all B-cells would be depleted, while, without being bound by theory, an anti-BCMA antibody therapy may not necessarily deplete all healthy B-cells of a patient, since BCMA is not or essentially not expressed on each and every healthy B-cell during development and/or homeostasis.

Thus, in a first aspect the preset invention provides a method for the stratification of a multiple myeloma (MM) patient disposed to respond favorably to an anti-BCMA antibody therapy, comprising determining whether B-cells, preferably malignant B-cells of said patient expresses BCMA protein on the surface of said B-cells, wherein said patient is disposed to respond favorably to said anti-BCMA antibody therapy, if said B-cells express BCMA protein on their surface.

In addition or in the alternative to the above described aspect, the present specification provides a method for diagnosing a BCMA negative multiple myeloma (MM) patient, comprising determining whether B-cells, preferably malignant B-cells of said patient express BCMA protein on their surface, wherein said patient suffers from BCMA negative MM, if no BCMA protein is detectable on the surface of said B-cells.

The term "stratification" as used herein includes the statistic division of a population of MM patients into subpopulation of MM patients with BCMA on the basis of expression of transcripts of BCMA gene, i.e. mRNA as well as of detection of said translated gene product, i.e. BCMA protein localized on the cell surface, for example, by an anti-BCMA antibody in order to consider whether or not a therapy using an anti-BCMA antibody for MM patients may be beneficial. Detection of BCMA on the cell surface of B-cells, preferably malignant B-cells is preferred. However, nevertheless, BCMA expression on mRNA level can be additionally tested/determined. Also, said term refers to sorting patients into those who may or may not benefit from an anti-BCMA antibody based therapy for treating or ameliorating multiple myeloma. In particular, stratifying patients involves determining as to whether not BCMA is expressed on the surface of B-cells, preferably malignant B-cells of patients suffering from MM. Those patients whose B cells express BCMA on the cell surface of B cells may benefit from said therapy, while those whose B cells do not express BCMA on the cell surface may not benefit from said anti-BCMA antibody therapy. However, such BCMA negative patients may then benefit from, for example, an anti-CD20, anti-CD38 and/or anti-CS1 antibody therapy or any other antibody therapy applied for the treatment or amelioration of MM as described herein.

The term "B-cells" used herein means lymphocytes that play an important role in the humoral immune response (as opposed to the cell-mediated immune response, which is governed by T cells). The principal functions of B cells are to make antibodies against antigens, perform the role of antigen-presenting cells (APCs) and eventually develop into memory B cells after activation by antigen interaction. In that stage (antibody production) B-cells include "plasma cells", sometimes also called herein "plasma B-cells". These plasma cells or plasma B-cells are also encompassed by the term "B cells" when used herein. However, also precursor B-cells as well as B-cells in all stages of their development and/or lineage commitment are encompassed by the term "B cells". B cells are an essential component of the adaptive immune system. While the term "B-cells" also includes normal (i.e., healthy) B-cells, it preferably includes malignant B-cells, in particular malignant B-cells associated with MM (i.e., MM B-cells).

Thus, in the context of MM patient the term "B cells" include both normal and malignant B-cells; with malignant B-cells being preferred (i.e., MM B-cells). "Malignant" describes lymphocytes (in particular B cells) that contribute to a progressively worsening disease, in particular MM as described herein. The term is most familiar as a description of cancer, here MM. Malignant BCMA positive B cells are not self-limited in their growth, are capable of invading into adjacent tissues, and may be capable of spreading to distant tissues (metastasizing). Malignant when used herein is synonymous with cancerous. These malignant B-cells are of main interest in the context of the present invention, since they should be targeted by antibodies, in particular by anti-BCMA antibodies, for which purpose these B-cells are analyzed for the expression of BCMA on their cell surface as described herein. MM patients that are subject to the methods and/or antibody-based therapies of the present invention have preferably malignant B-cells; said malignant B-cells are preferably Multiple myeloma cells. Of course, said patients also (or still have) have normal B-cells. Malignant B-cells can preferably be determined, detected and/or isolated by the means and methods described herein below. Briefly, malignant B-cells of a patient can be determined, detected and/or isolated by one or more of the specific (i.e., characteristic) B-cell surface marker(s) as described herein and, in particular as embodied in the claims (CD38 positive, CD56 positive or negative, CD 45 positive and/or CD19 positive).

The term, "Multiple Myeloma (MM)", also known as plasma cell myeloma or Kahler's disease (after Otto Kahler), is an incurable clonal B-cell neoplasia characterized by the accumulation of malignant plasma B-cells within the bone marrow, in close contact with stromal cells. MM is a progressive disease *inter alia,* for example, caused by multiple genetic insults, i.e. chromosomal translocations mainly caused by translocations such as t(11;14), t(4;14), t(8;14), or deletions such as del(13), and del(17) to the precursor plasma B-cell that tumor cells proliferate drastically and become apoptosis resistance.

B lymphocytes start in the bone marrow and move to the lymph nodes. As they progress, they mature and display different proteins on their cell surface. When they are activated to secrete antibodies, they are known as plasma cells. Multiple myeloma develops in B lymphocytes after they have left the part of the lymph node known as the germinal center.

The immune system keeps the proliferation of B cells and the secretion of antibodies under tight control. When chromosomes and genes are damaged, often through rearrangement, this control is lost. Often, a promoter gene moves (or translocates) to a chromosome where it stimulates an antibody gene to overproduction.

As mentioned above, a chromosomal translocation between the immunoglobulin heavy chain gene (on the fourteenth chromosome, locus 14q32) and an oncogene (often 11 q13, 4p16.3, 6p21, 16q23 and 20q11[10]) is frequently observed in patients with multiple myeloma. This mutation results in dysregulation of the oncogene which is thought to be an important initiating event in the pathogenesis of myeloma. The result is proliferation of a plasma cell clone and genomic instability that leads to further mutations and translocations. The chromosome 14 abnormality is observed in about 50% of all cases of myeloma. Deletion of (parts of) the thirteenth chromosome is also observed in about 50% of cases.

Accordingly, MM patients that are subject to the methods and/or antibody-based therapies of the present invention have preferably any of the herein mentioned chromosomal translocation(s) and/or deletion(s).
Production of cytokines (especially IL-6) by the plasma cells causes much of their localized damage, such as osteoporosis, and creates a microenvironment in which the malignant cells thrive. Angiogenesis (the attraction of new blood vessels) is increased. The produced antibodies are deposited in various organs, leading to renal failure, polyneuropathy and various other myeloma-associated symptoms.
In the context of the present invention, MM is preferably staged in accordance with the International Staging System (Greipp et al. (2005), J. Clin. Oncol. 23 (15):3412-3420 and/or in accordance with the Durie-Salmon Staging System (Durie et al. (1975), Cancer 36 (3): 842-854.

### International Staging System:

- Stage I: β₂-microglobulin (β2M) < 3.5 mg/L, albumin >= 3.5 g/dL
- Stage II: β2M < 3.5 mg/L and albumin < 3.5 g/dL; or β2M 3.5 mg/L - 5.5 mg/L irrespective of the serum albumin
- Stage III: β2M >= 5.5 mg/L

### Durie-Salmon staging system:

- stage I: all of
   o Hb > 10g/dL
   o normal calcium
   o Skeletal survey: normal or single plasmacytoma or osteoporosis
   o Serum paraprotein level < 5 g/dL if IgG, < 3 g/dL if IgA
   o Urinary light chain excretion < 4 g/24h
- stage II: fulfilling the criteria of neither I nor III
- stage III: one or more of
   o Hb < 8.5g/dL
   o high calcium > 12 mg/dL
   o Skeletal survey: Three or more lytic bone lesions
   o Serum paraprotein > 7g/dL if IgG, > 5 g/dL if IgA
   o Urinary light chain excretion > 12g/24h
   Stages I, II, and III of the Durie-Salmon staging system can be divided into A or B depending on serum creatinine:
   - A: serum creatinine < 2 mg/dL (< 177 umol/L)
   - B: serum creatinine > 2 mg/dL (> 177 umol/L)

Accordingly, MM patients that are subject to the methods and/or antibody-based therapies of the present invention are preferably staged in accordance with the International Staging System and/or in accordance with the Durie-Salmon Staging System.

Also, MM patients that are subject to the methods and/or antibody-based therapies of the present invention may have stage I, II or III MM in accordance with the International Staging System and/or in accordance with the Durie-Salmon Staging System.

MM patients may experience a variety of disease-related symptoms because of bony destruction, bone marrow infiltration, renal failure, immunodeficiency, and the psychosocial burden of a cancer diagnosis. Exciting new therapies and treatment approaches are becoming available but often bring unwanted side effects.
Most cases of myeloma also feature the production of a paraprotein, an abnormal antibody that can cause kidney problems and interferes with the production of normal antibodies leading to immunodeficiency. Hypercalcemia (high calcium levels) are often encountered. Myeloma is diagnosed with blood tests (such as protein electrophoresis, peripheral blood smear), microscopic examination of the bone marrow (bone marrow biopsy), and X-rays of commonly involved bones

Because many organs can be affected by myeloma, the symptoms and signs vary greatly. A mnemonic sometimes used to remember the common tetrad of multiple myeloma is CRAB: C = Calcium (elevated), R = Renal failure, A = Anemia, B = Bone renal failure, anemia, neurological symptoms.

The term "BCMA" when used herein encompass native sequence BCMA and BCMA variants (which are further defined herein). The BCMA may be isolated from a variety of sources, such as from murine or human tissue types or from another source, or prepared by recombinant or synthetic methods. "BCMA" is the abbreviation of B cell maturation antigen. BCMA was isolated as a receptor for BLyS (B Lymphocyte stimulator; trademark of Human Genome Science Rockville, MD), also referred to as BAFF, THANK, TALL-1, TNFSF13, zTNF4.

BCMA is type I single transmembrane receptors and belongs to the TNF family receptors, and is predominantly expressed on B lymphocytes. BCMA used herein also encompasses native sequence BCMA and BCMA variants (which are further defined herein), and may be isolated from a variety of sources, such as from murine or human tissue types or from another source, or prepared by recombinant or synthetic method.

A "native sequence BCMA comprises a polypeptide having the same amino acid sequence as BCMA derived from nature. Such native sequence BCMA can be isolated from nature or can be produce by recombinant or synthetic means. The naturally-occurring truncated or secreted forms of the BCMA (e.g. soluble forms containing for instance, an extracellular domain sequence), naturally-occurring variant forms (e.g., alternatively spliced forms) and naturally occurring allelic variants of the BCMA. The native sequence BCMA is a mature or full-length native sequence BCMA polypeptide comprising amino acids 1 to 184 of SEQ ID NO:1 or fragment thereof. Such fragments are preferably biologically active.

"Biologically active" as used herein, means having an in vivo or in vitro activity which may be performed directly or indirectly. Biologically active fragments of BCMA may have, for example, 70% amino acid homology with the active site of the receptor, more preferably at least 80%, and most preferably, at least 90% homology. Identity or homology with respect to the receptor is defined herein as the percentage of amino acid residues in the candidate sequence which are identical to the BCMA residues in SEQ ID NO:1, or which are identical to a defined portion of the amino acid residues in SEQ ID NO:1. Biological activity can be tested by way of interaction with APRIL and/or BAFF.

The "BCMA extracellular domain" or "BCMA ECD" refers to a form of BCMA which is essentially free of transmembrane and cytoplasmic domains of BCMA. Ordinarily, BCMA extracellular domain will have less than 1% of such transmembrane and cytoplasmic domains and will preferably have less than 0.5% of such domains. Optionally, BCMA ECD will comprise amino acid residues 8 to 41 of SEQ ID NO:1, or amino acid residues 4 to 51 of SEQ ID NO: 1, or amino acid residues 1 to 53 of SEQ ID NO:1. The BCMA ECD comprises amino acid residues 1 to 51 of SEQ ID NO:1. It will be understood by the skilled artisan that the transmembrane domain identified for the BCMA polypeptide is identified pursuant to criteria routinely employed in the art for identifying that type of hydrophobic domain. The exact boundaries of a transmembrane domain may vary but most likely by no more than about 5 amino acids at either end of the domain specifically mentioned herein. Accordingly, the BCMA ECD may optionally comprise amino acids 8-41 (SEQ ID NO: 1).

"BCMA-variant" means an active BCMA as defined below having at least about 80% amino acid sequence identity with the BCMA having the deduced amino acid sequence shown in SEQ ID NO:1 for a full-length native sequence BCMA or with a BCMA sequence. Such BCMA variants include, for instance, BCMA polypeptides wherein one or more amino acid residues are added, or deleted, at the end or C-terminus of the sequence of SEQ ID NO:1. Ordinarily, a BCMA variant will have at least about 80% or 85% amino acid sequence identity, more preferably at least about 90% amino acid sequence identity, and even more preferably at least about 95% amino acid sequence identity with the amino acid sequence of SEQ ID NO:1.

"Percent (%) amino acid sequence identity" with respect BCMA sequences identified herein is defined as the percentage of amino acid residues in a candidate sequence that are identical with the amino acid residues in the BCMA sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared.

The term "disposed to respond favorably" when used in the context of the present invention means that MM B-cells from a patient who is administered an antibody-based therapy, in particular an anti-BCMA antibody therapy are more likely to be susceptible to said antibody. The likelihood that MM B-cell (preferably obtained from a patient) may respond favorably is dependent on the expression of BCMA on the cell surface of a MM B-cell as described herein. More specifically, the MM B-cell may respond favorably to said antibody if expression level of BCMA on the cell surface of a MM B-cell is higher than that of (a) reference cell(s) as described herein. The expression level of BCMA on the cell surface of a MM B-cell is determined as described herein.

Surface expression of BCMA can, for example, be done by Fluorescence Activated Cell Sorting (FACS) using an appropriate anti-BCMA antibody. Anti-BCMA antibody can either be generated by means and methods commonly known in the art or are commercially available.

In particular, the specification also includes antibodies specifically reactive with BCMA. Antiprotein/anti-peptide antisera or monoclonal antibodies can be made by standard protocols (See, for example, Antibodies: A Laboratory Manual ed. by Harlow and Lane (Cold Spring Harbor Press: 1988)). A mammal such as a mouse, a hamster or rabbit can be immunized with an immunogenic form of the peptide. Techniques for conferring immunogenicity on a protein or peptide include conjugation to carriers, or other techniques, well known in the art. An immunogenic portion of BCMA can be administered in the presence of an adjuvant. The progress of immunization can be monitored by detection of antibody titers in plasma or serum. Standard ELISA or other immunoassays can be used with the immunogen as antigen to assess the levels of antibodies.

The antibodies are immunospecific for antigenic determinants of BCMA or its co-receptors, e.g. antigenic determinants of a polypeptide of SEQ ID NO: 1, or a closely related human or non-human mammalian homolog (e.g. 70, 80 or 90 percent homologous, more preferably at least 95 percent homologous). In yet a further preferred example, the anti-BCMA antibodies do not substantially cross react (i.e. react specifically) with a protein which is e.g., less than 80 percent homologous to SEQ ID NO:1; preferably less than 90 percent homologous with SEQ ID NO: 1; and, most preferably less than 95 percent homologous with SEQ ID NO:1. By "not substantially cross react", it is meant that the antibody has a binding affinity for a non-homologous protein which is less than 10 percent, more preferably less than 5 percent, and even more preferably less than 1 percent, of the binding affinity for a protein of SEQ ID NO:1.

As mentioned above, BCMA contains an extracellular domain. Said extracellular domain is preferably targeted by an antibody applied in the methods and antibody-based therapies of the present invention. A preferred anti-BCMA antibody is an anti-human BCMA antibody such as a rat anti-human BCMA antibody, a mouse anti-human BCMA antibody, a camelid anti-human BCMA antibody, a sheep anti-human BCMA antibody. Of course, if BCMA from a mammal other than human is to be detected on the surface of a patient, for example, a cat or dog, an antibody from a species other than that to be diagnosed is applied. For example, if the patient is a dog or cat, a mouse, rat, sheep, camelid anti-dog or anti-cat BCMA antibody is applied.

In the alternative to an anti-BCMA antibody also an anticalin, i.e., a lipocalin mutein such as a tear lipocalin mutein, bacterial lipocalin mutein or an hNGAL lipocalin mutein directed against BCMA, preferably human BCMA, can be applied.

A particularly preferred antibody applied in the methods of the present invention is the anti-human antibody Vicky-1 and/or MAB 193 (clone 335004). Vicky-1 is a rat IgG1 isotype and is available from GeneTex, Catalogue number #GTX17323. MAB193 is a rat IgG2a isotye and is available from R&D Systems, catalogue number #MAB193. Accordingly, in the methods of the present invention it is preferred that BCMA, preferably BCMA expressed on the surface of MM B-cells is detectable with Vicky-1 or MAB 193. Similarly, it is preferred that patients as described herein that may suffer from MM or are suspected to suffer therefrom have MM B-cells that are detectable with Vicky-1 or MAB 193. Put it differently, it is preferred that said patient (more specifically B-cells, in particular, MM B-cells of said patients) are, so to say, Vicky-1 and/or MAB193 positive.

Surface expression of BCMA can also be tested by a surface plasmon resonance (SPR) technique such as Biacore.

In the context of the methods of the present invention, a MM B-cell is deemed to express BCMA on its surface (i.e., it is BCMA positive or has a certain BCMA expression level), if it shows a detectable signal that is above (or exceeds that of) a reference cell, preferably a BCMA negative cell, more preferably a BCMA negative B-cell, even more preferably a BCMA negative MM B-cell. A preferred BCMA negative MM B-cell is U266B1, JJN-3 or LP-1, with U266B1 and JJN-3 being preferred. Notably, such a BCMA negative cell line may nevertheless have detectable BCMA mRNA. The skilled person is readily in a position to determine whether a detectable signal due to expression of BCMA on a B-cell of interest is above the detectable signal of a reference cell. "Above" in the context of a detectable signal means the detectable signal due to expression of BCMA on the surface of a B-cell of interest such as one or more B-cells from a patient, is 5, 10, 15, 20, 25, 30, 35, 40, 45, 50, 55, 60, 65, 70, 75, 80, 85, 90, 95, 100% or higher than the signal from a BCMA negative reference cell. As mentioned above, the MAB193 or Vicky-1 antibody is preferably used for detecting/isolating/determining BCMA expression on the surface of B-cells, preferably malignant B-cells of a patient, preferably MM patient.

A "signal" or "detectable signal" reflects the result of an antibody-antigen reaction. In the context of the present invention the antibody-antigen reaction is preferably the reaction of an anti-BCMA antibody with BCMA.

A signal can be determined by way of the signal provided by the signal generating group of an antibody, in particular an antibody against BCMA which is described herein. The signal can be any signal which is detectable by, for example, spectroscopic, photochemical, biochemical, immunochemical, or chemical means as described herein below. A signal can also be determined by way of the signal provided by the signal generating group of an antigen-specific receptor which is described herein below.

A signal generating group refers to a composition detectable by spectroscopic, photochemical, biochemical, immunochemical, or chemical means. For example, useful labels include radiolabels such as ³²P, ³⁵S, or ¹²⁵I; fluorescent dyes (for example, Cy-3, Cy-5); chromophores, electron-dense reagents; enzymes that generate a detectable signal (e.g., as commonly used in an ELISA); or spin labels. The label or detectable moiety has or generates a measurable signal, such as a radioactive, chromogenic, or fluorescent signal, that can be used to quantify the amount of bound detectable moiety in a sample.

The detectable moiety can be incorporated in or attached to signal generating group either covalently, or through ionic, van der Waals or hydrogen bonds, e.g., incorporation of radioactive nucleotides, or biotinylated nucleotides that are recognized by streptavidin. The label or detectable moiety may be directly or indirectly detectable. Indirect detection can involve the binding of a second directly or indirectly detectable moiety to the detectable moiety. For example, the detectable moiety can be the ligand of a binding partner, such as biotin, which is a binding partner for streptavidin, or a nucleotide sequence, which is the binding partner for a complementary sequence, to which it can specifically hybridize. The binding partner may itself be directly detectable, for example, an antibody may be itself labelled with a fluorescent molecule. The binding partner also may be indirectly detectable.

When the antibody-specific receptor is "capable of binding to a signal generating group" this means that it can bind to a signal generating group. For example, the antibody-specific receptor may carry a functional group which is able to bind to a signal generating group. A functional group can be streptavidin/avidin which binds to biotin, an antigen such as a tag, for example, GST or histidine residues which binds to an antibody, a sugar which binds a lectin or the known Dig/Anti-Dig system. The moiety that binds to the functional group carries the signal generating group.

Any immunoassay known in the art can be used for immunologically detecting the binding of an antibody to an antigen or the binding of an antigen to an antibody. Immunoassays are well known to the skilled artisan. Methods for carrying out such assays as well as practical applications and procedures are summarized in related textbooks. Examples of related textbooks are Tijssen, P., In: Practice and theory of enzyme immunoassays, Burdon, R.H. and v. Knippenberg, P. H. (eds.), Elsevier, Amsterdam (1990), pp. 221-278, and various volumes of Colowick, S. P. and Caplan, N.O. (eds.), Methods in Enzymology, Academic Press, dealing with immunological detection methods, especially volumes 70, 73, 74, 84, 92 and 121.

Immunoassays, for example, include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, solid phase immune tests, and mass spectrometry such as SELDI-TOF, MALDI-TOF, or capillary electrophoresis-mass spectrometry (CE-MS).
Furthermore, suitable immunoassays include microplate ELISA-based methods, fully-automated or robotic immunoassays and latex agglutination assays.

Typically, an immunoassay is an assay in which an antibody specifically binds an antigen to provide for the detection and/or quantification of an antibody or an assay in which an antigen binds to an antibody to provide for the detection and/or quantification of said antigen. The antigen is preferably BCMA or a fragment thereof, said fragment being preferably immunologically detectable, and the antibody is preferably an anti-BCMA antibody. "Immunologically detectable" when used herein preferably includes that a signal generated by the binding of an antibody to an antigen or by the binding of an antigen to an antibody as described herein is detectable by means and methods commonly known and applied in the art. In the context of the present, "immunologically detectable" preferably means that a signal generated by the reaction of an anti-BCMA antibody and BCMA or fragment thereof is above (or exceeds that of) a reference cell as described herein, i.e., a MM B-cell of interest is then BCMA positive.

"Is not immunologically detectable" when used herein preferably includes that a signal generated by the binding of an antibody to an antigen or by the binding of an antigen to an antibody as described herein is not detectable by means and methods commonly known and applied in the art. In the context of the present, "not immunologically detectable" preferably means that a signal generated by the reaction of an anti-BCMA antibody and BCMA or fragment thereof is equal to or below that of a reference cell as described herein, i.e., a MM B-cell of interest is then BCMA negative.

"MM B-cell of interest" means B-cells, preferably malignant B-cells obtained from a patient suffering from MM or who is suspected to suffer from MM. Prior to analyzing said MM B-cell of interest in accordance with the methods of the present invention, it cannot be said as to whether or not said B-cells are BCMA positive or negative, respectively.

In addition or as an the alternative to a comparison of the expression level of BCMA on the cell surface of a MM B-cell with a BCMA negative cell, also a BCMA positive cell line can serve as a reference cell. Suitable BCMA positive cell lines in the sense of the present invention (i.e., as defined herein) are NCI-H929, MOLP-2, OPM-2, MOLP-8, RPMI8226, KMS-12-BM or L-363. Specifically, a MM B-cell of interest is deemed to be BCMA-positive if it has at least the equal expression level of BCMA as a reference cell line. The expression is preferably determined as described herein (i.e., by way of the signal generated by a cell).

Similarly, In the context of the methods of the present invention, a MM B-cell is deemed to not express BCMA on its surface (i.e., it is BCMA negative), if it does essentially not show a detectable signal that is above a reference cell, preferably a BCMA negative cell, more preferably a BCMA negative B-cell, even more preferably a BCMA negative MM B-cell. A preferred BCMA negative MM B-cell is U266B1, JJN-3 or LP-1, with U266B1 and JJN-3 being preferred. Notably, such a BCMA negative cell line may nevertheless have detectable BCMA mRNA. The skilled person is readily in a position to determine whether a detectable signal due to expression of BCMA on a B-cell of interest is not above (i.e., equal to or below) the signal of a reference cell.
"Equal to" in the context of a detectable signal means the detectable signal due to expression of BCMA on the surface of a B-cell of interest such as one or more B-cells from a patient is the same as the signal of a BCMA negative reference cell.
"Below" in the context of a detectable signal means that the B-cell of interest such as one or more B-cells from a patient shows a signal that is 5, 10, 15, 20, 25, 30, 35, 40, 45, 50, 55, 60, 65, 70, 75, 80, 85, 90, 95 or even 100% lower than the signal from a BCMA negative reference cell.

The findings of the present inventors also provide the skilled person with a method for selecting an antibody-based multiple myeloma (MM) therapy, comprising determining whether B-cells of a patient express BCMA protein on the surface of said plasma B-cells, wherein, if said plasma B-cells are BCMA positive, the patient may be subject to an anti-CD20 antibody therapy and/or an anti-CD38 antibody therapy and/or an anti-BCMA antibody therapy, or, if said B-cells are BCMA-negative, the patient is subject to an anti-CD20 antibody therapy and/or an anti-CD38 antibody therapy.

Indeed, as described herein, if B-cells of a patient express BCMA protein, said patient will potentially respond favourably to an anti-BCMA antibody therapy. However, even if B-cells of a patient are negative for BCMA (BCMA-negative), said patient can be subject to an antibody-based therapy such as an anti-CD20 antibody-based therapy and/or anti-CD38 antibody based therapy. Thus, the present invention provides methods and means for selecting one or more appropriate antibody-based therapies for the treatment or prevention of MM.

The term "potentially" when used in the context of a therapeutic effect means that an anti-BCMA antibody- though such an antibody is deemed to have a therapeutic effect based on the outcome of the methods of the present invention - does not necessarily have to be therapeutically effective. This is so because - self-explanatory as it is - the methods of the present invention cannot provide a 100% safe prediction whether or not a patient may be susceptible to such an antibody, since, apart from the expression of BCMA on the surface of a MM B-cell, individual factors such as age, body weight, general health, sex, diet, drug interaction and the like may have an influence as to whether or not a patient will be susceptible to such an antibody.
However, if a MM B-cell from a patient is BCMA positive as described herein, the likelihood that an anti-BCMA antibody has a therapeutic effect is more than 50% in comparison to a patient, whose MM B-cells does not show cell surface expression of BCMA. Preferably the likelihood is more that 60%, 70%, 80% or 90%, more preferably more than 95%.

Of course, it is advisable to determine as to whether a BCMA-negative MM patient may be CD20 and/or CD38-positive and/or CS1-positive before making him/her subject to an anti-CD20 and/or anti-CD38 and/or CS1 antibody-based therapy. Further antibody-based therapies (i.e., one or more antibody-based therapies) in addition (i.e., as combination) to an anti-BCMA antibody-based therapy (if a patient is BCMA positive) or as an alternative to an anti-BCMA antibody-based therapy (if a patient is BCMA negative), may be based on the target indicated in the outer left column of the table that follows below. If the antibody directed against the target as indicated in the outer left column is conjugated to a toxin, then such a conjugate is also encompassed by said further antibody-based therapy.

| **Target** | **Brand name** | **Type of mAb (conjugate)** | **Phase** | **Remarks** |
|---|---|---|---|---|
| CD138 | B-B4-DM1 | the maytansinoid immunoconjugate mouse IgG1 mAb B-B4 | preclinical | Tassone Blood 2004,104:3688-96 |
| HM1.24 | humanized HM1.24 | humanized | preclincial | Ozaki Blood 1999,93:3922-3930 |
| | humanized HM1.24 | Fc-engineered humanized IgG₁ | preclincial | |
| IL-6 | OP-R003-1, 1339 Elsilimomab, Azintrel | human IgG1 | preclincial | Fulciniti Clin Cancer Res 2009,15:7144-52 |
| HLA-DR | 1D09C3 | human IgG1 | preclincial | Carlo-Stella Cancer Res 2007 |
| kininogen | C11C1 | mouse | preclincial | Sainz Cancer Immunol Immunother 2006 C11C1 mAb inhibits its own tumor growth *in vivo,* slows down B38-MM growth rate when both MM are implanted together and when mAb C11C1 is injected intraperitoneally. MAb C11C1-treated-MM showed decreased MVD and kininogen binding *in vivo* without FGF-2, B1R, or B2R expression changes |
| HLA class I | 2D7-DB | converted from mouse IgG2b, single-chain Fv diabody | preclincial | Sekimoto Cancer Res 2007; 67:1184-92. a recombinant single-chain Fv diabody 2D7-DB specifically induces multiple myeloma cell death in the bone marrow environment |
| *β*2-microglobulin | anti-*β*2M mAbs | mouse | preclincial | Yang Blood 2007; 110:3028-35. & Clin Cancer Res 2009; 15:951-9. Strong apoptotic effect on myeloma cells and low toxocity in the mice suppports potential use as therapeutic agents |
| CD32B | MGA321(2B6) | humanized IgG1 | preclincial | Zhou Blood 2008; 111:549-557. humanized 2B6 MoAb may target in patients with systemic AL-amyloidosis. It blocks Fc engagement of CD32B and may improve the performance of other cancer Mabs when combined with them during administration |
| FGFR3 | PRO-001 | human IgG1 | preclincial | Trudel Blood 2006; 2:4908-4915. The inhibitory anti-FGFR3 antibody, PRO-001, is cytotoxic to t(4;14) MM cells and deserves further study for the treatment of FGFR3-expressing myeloma |
| ICAM-1 | cUV3 | chimeric IgG1 | preclincial | Smallshaw J Immunother 2004; Coleman J Immunother 2006 cUV3 significantly prolongs the survival of SCID/ARH-77 mice |
| BLyS | BLyS/rGel | Fusion protein of an antibody tethered to a toxin | preclincial | Lyu et al. Mol Cancer Ther 2007; 6:460-70 |
| TACI | Atacicept (TACI-Ig) | fusion protein | preclincial | Yaccoby Leukemia 2008 22, 406-413 |
| CD70 | SGN-70 | humanized IgG1 | preclincial | McEarchern Clin Cancer Res 2008 14, 7763-72 |
| TRAIL-R2(DR5) | lexatumumab | human | preclincial | Menoret et al. Blood 2006; 132: 1356-62 |
| IL-6R | NRI (engineered Tocilizumab) | a single-chain fragment format dimerized by fusing to the Fc portion of human immunoglobulin G1 | preclincial | Yoshio-Hoshino Cancer Res 2007; 67:871-5. the NRI gene introduction combined with adenovirus gene delivery inhibited the *in vivo* S6B45 cell growth significantly |
| BCMA | SG1 | Auristatin-BCMA mAb | preclincial | Ryan et al. Mol Cancer Ther 2007; 6:3009-18 |
| matriptase | M24-DOX | immunoconjugate with doxorubicin | preclincial | Bertino et al. 2010 AACR abtract no. 2596. M24-DOX is as potent as free doxorubicin to inhibit the growth of MM cells. But target delivery of doxorubicin by the matriptase antibody significantly reduced the toxicity toward cardiomyocytes that lack matriptase expression |
| IL-1beta | XOMA 052 | Human Engineered IgG2 | preclincial | Lust 2010 AACR abstract no. 2449. XOMA 052 is highly effective at inhibiting IL-1 induced IL-6 production in myeloma patients *in vitro* |
| CD20 | Rituxan | chimeric with a human IgG1 Fc | II (ongoing) | NCT00258206 (with cyclophosphamide): NCT00505895. High-dose cyclophosphamide in combination with rituximab in patients with primary refractory, high-risk, or relapsed myeloma, also being studied for the treatment of peripheral neuropathy in patients with MGUS |
| CD20 | Zevalin (yttrium Y 90 ibritumomab tiuxetan) | mouse IgG1 | I (ongoing) | NCT00477815: Zevalin radioimmunotherapy with high-dose melphalan and stem cell transplant for MM |
| CD40 | SGN-40 (Dacetuzumab) | humanized IgG1 | Ib (ongoing) | NCT00664898: safety and pharmacology of SGN-40 administered in combination with Bortezomib (Velcade, PS-341) in patients with relapsed or refractory MM. NCT00525447 is the study of SGN40, lenalidomide, and dex in MM patients |
| CD40 | HCD122 (Lucatumumab) | human IgG1 | I (ongoing) | NCT00231166 Dose-finding trial of HCD122 in MM patients that is relapsed or has not responded to prior therapy |
| CD20 | Bexxar(131-tositumomab) | radioactive iodine 131 attaching to anti-CD20; muIgG2a (131) | II (ongoing) | NCT00135200: to see whether the treatment with Bexxar will decrease and possibly eliminate residual myeloma cells resistant to chemotherapy |
| CD56 | BB-10901 (IMGN901) | humanized (maytansine DM1 conjugation) | I (ongoing) | NCT00346255: given as an intravenous infusion weekly for two consecutive weeks every three weeks to relapsed and relapsed refractory CD56-positive MM; NCT00991562: IMGN901 in combination with lenalidomide and dexamethasone |
| RANKL | Denosumab | human IgG2 | II/III (ongoing) | NCT00259740: to determine if denosumab is effective in the treatment of relapsed or plateau-phase MM; NCT00104650: to determine the effectiveness of AMG 162 in reducing urinary N-telopeptide in advanced cancer subjects with bone metastases; NCT00330759: Phase III Study of Denosumab Compared With Zoledronic Acid (Zometa) in the Treatment of Bone Metastases in Subjects With Advanced Cancer (Excluding Breast and Prostate Cancer) or MM |
| VEGF | Avastin beuacizumab | humanized | II (ongoing) | NCT00428545 (in combination with bortezomib); NCT00410605 (added with lenalidomide and dexamethasone) |
| CD52 | Campath-1H (alemtuzumab) | humanized | II (ongoing) | NCT00625144: studying the side effects of giving fludarabine and busulfan together with alemtuzumab followed by donor stem cell transplant and to see how well it works in treating patients with hematological cancer or other disease |
| IL-6 | CNTO 328 | chimerized IgG1 | I/II (ongoing) | NCT00401843 (in combination with bortezomib); NCT00911859 (added with Velcade-Melphalan-Prednisone); NCT00402181 (in combination with dexamethason) |
| IL-6 | B-E8 (Elsilimomab) | murine | II | Preliminary efficacy was seen but there is a limitation for the clinical use of a murine monoclonal antibody since it frequently induces human antimouse antibodies (HAMA) |
| IL-6R | MRA (Tocilizumab) | humanized | II | |
| TRAIL-R1(DR4) | Mapatumumab(TRM-1) | human | II (ongoing) | NCT00315757 (in combination with bortezomib) |
| EGFR | Erbitux(EMMA-1) | chimerized | II (ongoing) | NCT00368121 (in combination with dexamethasone) |
| CS1 | elotuzumab/HuLuc63 | humanized | I/II (ongoing) | NCT00742560 & NCT00726869 (in combination with bortezomib) |
| DKK | BHQ880 | human IgG1 | I/II (ongoing) | NCT00741377: in combination with Zoledronic Acid in relapsed/refractory myeloma |
| CD138 | BT062 | chimeric (B-B4-maytansinoid DM4) | I (ongoing) | NCT00723359 |
| the activin receptor type IIA (ActRIIA) | ACE-011 | human IgG1 | I/IIa (ongoing) | NCT00747123 (in patients with osteolytic lesions with MM) |
| IGF-1R | AVE1642 | humanized | I/II (ongoing) | Descamps et al. (B J Cancer 2009; 100:366) Anti-IGF-1R Monoclonal Antibody combined with bortezomib for patients with rel/ref MM |
| Ganglioside GM2 | BIW-8962 | humanized | I/II (ongoing) | Dosing study of anti-GM-2 ganglioside (expressed at high levels on the surface of MM cells) followed by efficacy study |
| CD74 (variant MHC II) | milatuzumab (hLL1, IMMU-110) | humanized IgG1 or humanized IgG1 doxorubicin conjugate | I/II (ongoing) | NCT00421525: in patients with recurrent or refractory multiple myeloma who have failed at least two prior standard systemic treatments. Its isotope, drug, and toxin conjugates have high antitumor activity in non-Hodgkin's lymphoma and multiple myeloma *in vitro* and in tumor xenograft models. Stein et al. 2007 & 2009 |
| Alpha-4 integrin | natalizumab (Tysabri) | humanized IgG4 | I/II (ongoing) | NCT00675428: patients with relapsed or refractory multiple myeloma |
| MHC II (HLA-DR) | 1D09C3 | human IgG4 | I | Carlo-Stella et al. 2007 showed that IFN-gamma-induced up-regulation of HLA-DR results in a potent enhancement of the *in vivo* antimyeloma activity of 1D09C3 in mice. Initial clinical testing with 1D09C3 has not raised any unexpected or unacceptable safety concerns and the maximum tolerated dose has not yet been reached. GPC Biotech has decided to not put further internal resources into developing 1D09C3 due to potential swapping of IgG4 antibody one half of its Y-shaped structure with the half of a different antibody, thus resulting in a new molecule whose properties are unknown. However, the Company will seek a partner for the intellectual property relating to this program |
| IGF-1R | CP-751,871/figitumumab | human IgG2 | I | Lacy et al. (J. Clin Onclo 26:3196) reported that CP-751,871 is well tolerated and may constitute a novel agent in the treatment of multiple myeloma |
| KIR | IPH 2101 | human IgG4 | I/IIa (ongoing) | NCT00552396 (ASCO May 30 2009 abstract 09-AB-3032) safety and tolerability study for patients with relapsed/refractory MM. Preclinical characterization of 1-7F9, a novel human anti-KIR therapeutic antibody that augments NK-mediated killing of tumor cells (Romagne et al. 2009) |

B-cells can be selected and/or identified by way of a B-cell surface marker. A "B cell surface marker" or "B cell surface antigen" herein is an antigen expressed on the surface of a B cell which can be targeted with an antagonist which binds thereto. Exemplary B cell surface markers include, but are not limited to CD19, CD20, CD21, CD22, CD23, CD24, CD37, CD40, CD52, D53, CD72, CD73, CD74, CDw75, CDw76, CD77, CDw78, CD79a, CD79b, CD80, CD81, CD82, CD83, CDw84, CD85, CD86, CDI 80 (RP105), FcRH2 (IRTA4), CD79A, C79B, CR2, CCR6, CD72, P2X5, HLA-DOB, CXCR5 (BLRI), FCER2, BR3 (aka BAFF-R), TACI, BTLA, NAG14 (aka LRRC4), SLGC16270 (aka LOC283663), FcRHI (IRTA5), FcRH5 (IRTA2), ATWD578 (aka MGC15619), FcRH3 (IRTA3), FcRH4 (IRTAI), FcRH6 (aka LOC343413) and, in particular BCMA.

MM B-cells may be detected, selected and/or identified by way of a B-cell surface marker as described above. However, preferably MM B-cells are selected and/or identified by way of the following markers: CD19, CD56, CD117, CD20, CD28, CD27, CD81 and/or CD200 (see also Table 2 of Rawston et al. (2008), Haematologica 93(3):431-438.
MM B-cells of the present invention are preferably characterized to be at least CD38 positive, CD56 positive or negative, CD45 positive, and CD19 positive.

B-cells, preferably malignant B-cells that are subject to the methods of the present invention are preferably obtained from a patient. A patient according to this invention is a mammal, preferably said patient or mammal suffers from MM or is suspected to suffer from MM. "Mammal" for purposes of treatment refers to any animal classified as a mammal, including human, domestic and farm animals, nonhuman primates, and any other animal that has mammary tissue. A mammal includes human, rodents such as mouse, rat or rabbit, dog, cat, chimpanzee, horse, pig, etc., with human being preferred. A patient also includes human and veterinary patients, with human patients being preferred. In each one of the above methods, the mammal may be one that suffers from multiple myeloma.
A mammal "in need" of treatment can include, but are not limited to, mammals that have multiple myeloma or are suspected to have multiple myeloma.

A "subject" when used herein includes mammalian and non-mammalian subjects.

B-cells obtained from a patient are preferably contained in a sample. In accordance with the present invention by the term "sample" is intended any biological sample obtained from a subject, cell line, tissue culture, or other source containing at least B-cells. Biological samples include body fluids (such as blood, serum, plasma, urine, saliva, synovial fluid and spinal fluid) and tissue sources found to contain B-cells. Methods for obtaining tissue biopsies and body fluids from subjects are well known in the art. Generally, a biological sample which includes peripheral blood mononuclear cells (PBMCs), in particular B cells and T cells is preferred as a source. A sample which includes peripheral blood mononuclear cells (PBMCs), in particular B cells and T cells is preferably taken from peripheral blood of a human patient. Other preferred samples are whole blood, serum, plasma or synovial fluid, with plasma or serum being most preferred. However, a sample from peripheral blood of a human patient is particularly preferred.

It is a preferred embodiment of the methods of the present invention that a patient is to be treated with an anti-BCMA antibody. The antibody applied for the treatment of a patient as well as the antibody applied in the methods for stratification, diagnosis or selecting an antibody-based MM therapy is preferably a monoclonal antibody, polyclonal antibody, chimeric antibody, humanized antibody, bispecific antibody, domain antibody (dAb) or nanobody.

The term "antibody" also includes but is not limited to polyclonal, monoclonal, monospecific, polyspecific such as bispecific, non-specific, humanized, human, single-chain, chimeric, synthetic, recombinant, hybrid, mutated, grafted, domain (VHH) and in vitro generated antibodies as well as nanobodies including camelid antibodies and humanized camelid antibodies. Accordingly, the term "antibody" also relates to a purified serum, i.e., a purified polyclonal serum. Accordingly, said term preferably relates to a serum, more preferably a polyclonal serum and most preferably to a purified (polyclonal) serum.
Furthermore, the term "antibody" as employed in the invention also relates to derivatives or variants of the antibodies described herein which display the same specificity as the described antibodies. Examples of "antibody variants" include humanized variants of non- human antibodies, "affinity matured" antibodies (see, e.g. Hawkins et al. J. Mol. Biol. 254, 889-896 (1992) and Lowman et al., Biochemistry 30, 10832- 10837 (1991)) and antibody mutants with altered effector function (s) (see, e.g., US Patent 5, 648, 260). The terms "antigen-binding domain", "antigen-binding fragment" and "antibody binding region" when used herein refer to a part of an antibody molecule that comprises amino acids responsible for the specific binding between antibody and antigen. The part of the antigen that is specifically recognized and bound by the antibody is referred to as the "epitope" as described herein above. As mentioned above, an antigen-binding domain may typically comprise an antibody light chain variable region (VL) and an antibody heavy chain variable region (VH); however, it does not have to comprise both. Fd fragments, for example, have two VH regions and often retain some antigen-binding function of the intact antigen-binding domain. Examples of antigen-binding fragments of an antibody include (1) a Fab fragment, a monovalent fragment having the VL, VH, CL and CH1 domains; (2) a F(ab')2 fragment, a bivalent fragment having two Fab fragments linked by a disulfide bridge at the hinge region; (3) a Fd fragment having the two VH and CH1 domains; (4) a Fv fragment having the VL and VH domains of a single arm of an antibody, (5) a dAb fragment (Ward et al., (1989) Nature 341 :544-546), which has a VH or VL domain; (6) an isolated complementarity determining region (CDR), and (7) a single chain Fv (scFv). Although the two domains of the Fv fragment, VL and VH> are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci USA 85:5879-5883). These antibody fragments are obtained using conventional techniques known to those with skill in the art, and the fragments are evaluated for function in the same manner as are intact antibodies.
The term "immunoglobulin" (Ig) is used interchangeably with "antibody" herein.

A further aspect of the present invention is an anti-BCMA antibody therapy for use in the treatment of a multiple myeloma (MM) patient whose plasma B-cells are disposed to be BCMA positive.

A still further aspect is an anti-BCMA antibody for use in the treatment of a multiple myeloma (MM) patient diagnosed with a method of the present invention.

Also another aspect is an anti-CD20 antibody and/or an anti-CD38 antibody for use in the treatment of a multiple myeloma (MM) patient whose B-cells are BCMA negative.

In one embodiment of the present invention, an anti-BCMA antibody is conjugated to a toxin including chemotherapeutic agent that is toxic against malignant plasma B-cells in MM patients. Suitable chemotherapeutic agents are disclosed in WO 2010/121093 such as Bortezomib (Velcade), Thalidomide (Thalomid), Lenalidomide (Revlimid).

The terms "treat" or "treatment" refer to both therapeutic treatment and prophylactic or preventative measures, wherein the object is to prevent or slow down (lessen) an undesired physiological change or disorder, such as the development or spread of cancer, in particular MM. For purposes of this invention, beneficial or desired clinical results include, but are not limited to, alleviation of symptoms, diminishment of extent of disease, stabilized (i. e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The anti-BCMA antibody, anti-CD20 antibody, anti-CD38 antibody and/or anti-CS1 antibody is preferably administered in an "effective amount". An "effective amount" is an amount sufficient to carry out a specifically stated purpose. An "effective amount" can be determined empirically and by known methods relating to the stated purpose. The term "therapeutically effective amount" refers to an amount of a therapeutic agent of this invention effective to "treat" or "ameliorate" MM in a mammal (aka patient). In one instance, the therapeutically effective amount may be a growth inhibitory amount or a cytotoxic amount In the case of MM, the therapeutically effective amount of the drug active for any one of the following: reducing the number of cancer cells; reducing the number of malignant MM B-cells, tumor size; inhibiting (i.e., slow to some extent and preferably stop) cancer cell infiltration into peripheral organs; inhibiting (i.e., slow to some extent and preferably stop) tumor metastasis; inhibiting, to some extent, tumor growth; and/or relieve to some extent one or more of the symptoms associated with the cancer. To the extent the drug can prevent growth and/or kill existing cancer cells, it can be cytostatic and/or cytotoxic. A "treatment" or a "therapeutically effective amount" refers to a course of administration of the therapeutic agent, which course may include several dosings spread over a period of time to achieve a desired effect.

The B cell depleting agents (i.e., the anti-BCMA antibody, anti-CD20 antibody, anti-CD38 antibody or any other antibody directed against a target expressed on the surface of a B cell such as CS1) can be administered to a patient by a variety of methods, such as by intravenous administration, e.g., as a bolus or by continuous infusion over a period of time, by subcutaneous, intramuscular, intraperitoneal, intracerobrospinal, intra-articular, intrasynovial, intrathecal, or inhalation routes.

These antibodies are preferably administered in the form of a pharmaceutical compositions. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition may comprise further agents such as additional anti-tumor agents as exemplified herein elsewhere.

Another embodiment of the invention is the use of a kit comprising an anti-BCMA antibody and instructions for use, in particular instructions as how to perform the methods of the present invention.
The kit comprises at least one container and a label or package insert on or associated with the container. Suitable containers include, for example, bottles, vials, syringes, etc. The containers may be formed from a variety of materials such as glass or plastic. The container can have a sterile access port for extracting a therapeutic agent (for example the container may be an intravenous solution bag or a vial having a stopper pierceable by a hypodermic injection needle). The label or package insert can indicate that the composition is used for treating MM.

Additionally, the kit may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes

### EXAMPLES

### ALEXA Fluor® 488 Labeling of antibodies

Anti-BCMA IgGs and appropriate isotype control IgGs were labeled with ALEXA Fluor® 488 using ALEXA Fluor® 488 Monoclonal Antibody Labeling Kit (Invitrogen, #A20181). Labeling and calculation of the degree of labeling was performed according to manufacturers' instructions.

**Table 1: anti-BCMA monoclonal antibodies used for ALEXA Fluor® 488 labeling**

| **IgG** | **Clone** | **Isotype** | **Cat. No.** |
|---|---|---|---|
| **Anti-BCMA** | Vicky-1 | Rat IgG1 | GeneTex, #GTX17323 |
| **Anti-BCMA** | 335004 | Rat IgG2a | R&D Systems, #MAB193 |
| **Rat IgG1 isotype ctrl.** | 43414 | Rat IgG1 | R&D Systems, #MAB005 |
| **Rat IgG2a isotype ctrl.** | 54447 | Rat IgG2a | R&D Systems, #MAB006 |

### Staining primary malignant plasma B-cells from bone marrow aspirates

Bone marrow aspirates from MM patients were filtered through a 70 µm cell strainer (BD, 352350). Plasma cell numbers have previously been determined and based on that, aspirate volumes for staining were calculated:
1-10% plasma cells in the CD45 negative population 180 µl;
11-20% plasma cells in the CD45 negative population 120 µl;
21-31% plasma cells in the CD45 negative population 60 µl;

An appropriate volume of the aspirates was incubated with 1.5 µl of each of the following antibodies: CD38-APC (BD, clone: HIT2, #555462), CD56-PE-Cy-7 (BD, clone: NCAM16.2, #335791), CD45-APC-Cy7 (BD, clone: 2D1; #557833) and 6µl of CD19-PerCP-Cy5.5 (BD, clone: HIB19, #561295). For diagnostic staining cells were fixed and permeabilised using Fixation and Permeabilisation kit (Invitrogen, #GAS-003) followed by 6 µl each of anti-Ig kappa-FITC (BD, clone: G20-193, # 555791) and anti-Ig lambda-PE (BD, clone: JDC-12,#555797). For detection of BCMA, 10 µg/ml Vicky-1-Alexa488 or MAB193-Alexa488 and of the isotype controls rat IgG1-Alexa488 and rat IgG2a-Alexa488 were used and samples were incubated for 15 min at 4 °C. Cells were washed and red blood cells lysed using a FACS Lyse/Wash Assistant, LWA (BD). Signals were analyzed using a FACSCanto flow cytometer (BD).

Plasma cells were identified by gating on the CD45 low, CD38 high, CD19 low population and, in case the patients expressed CD56 on their plasma cells, on CD56 high. As reference non plasma cells were gated on for CD45 high, CD38 low, CD56 low. The fluorescence intensities of the Alexa488 were measured for both populations, e.g. for Vicky-1 and rat IgG1. Histogrammes derived from specific antibody and isotype control fluorescent signals were overlaid and the difference noted. Patients are considered BCMA positive if their non-plasma cells show no difference in fluorescence intensity for Vicky-1 and Mab193 and isotype controls as well as a clear increase in fluorescence on plasma cells for Vicky-1 and MAB193 as compared to the fluorescence measured for rat IgG1 or IgG2a respectively.

Raw data was analyzed using Flow Jo. Patients were given consecutive numbers for identification in addition to their sample number assigned on the date of data collection.

### FACS staining of OPM-2 cells with different anti-BCMA antibodies

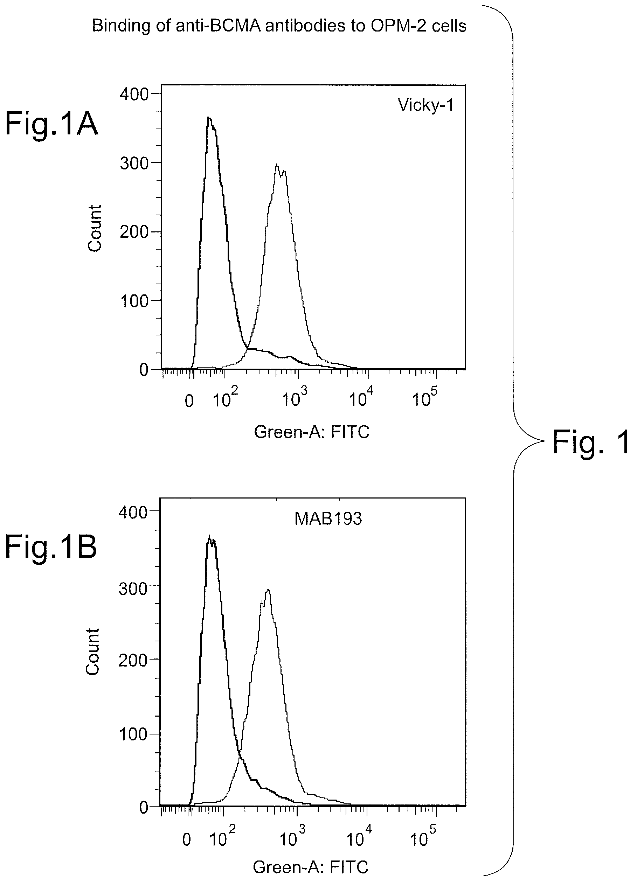

In order to examine whether or not BCMA protein is expressed on cell surface of OPM-2 cell line which is a human, peripheral blood derived from multiple myeloma cells, Vicky-1 as well as MAB193 were used for FACS analysis (Fig. 1). As a result, both showed sufficient staining efficacy for BCMA protein expressing in a surface of OPM-2 cells. However, it was found that Vicky-1 showed a stronger FACS shift compared to MAB193 (Fig. 1), which mean fluorescence signals of Vicky-1 and MAB193 also proved (Table 2). Therefore, Vicky-1 as anti-BCMA antibody was used for further experiments conducted herein.

**Table 2: Mean fluorescence signals**

| **mAb** | **Mean Fluorescence Signal** |
|---|---|
| **Vicky-1** | 609 |
| **MAB193** | 371 |

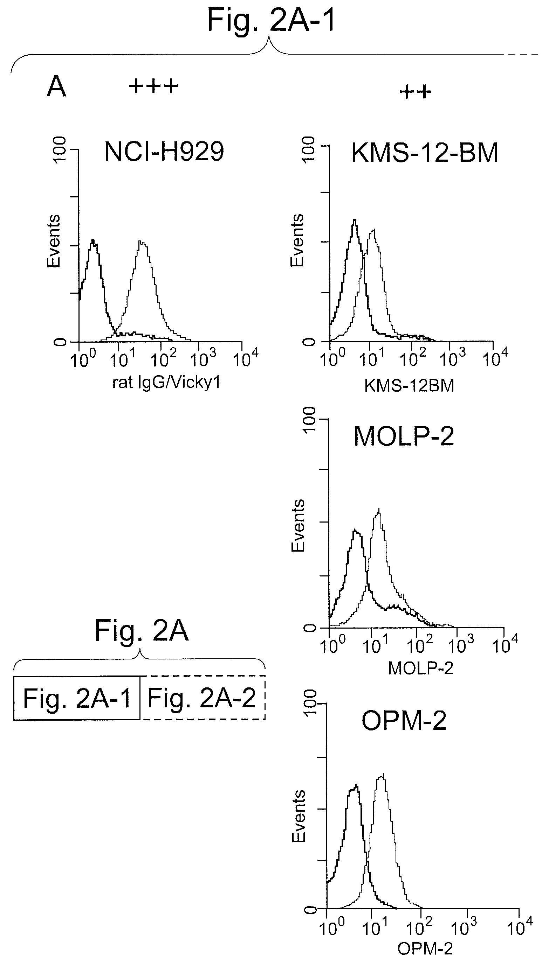

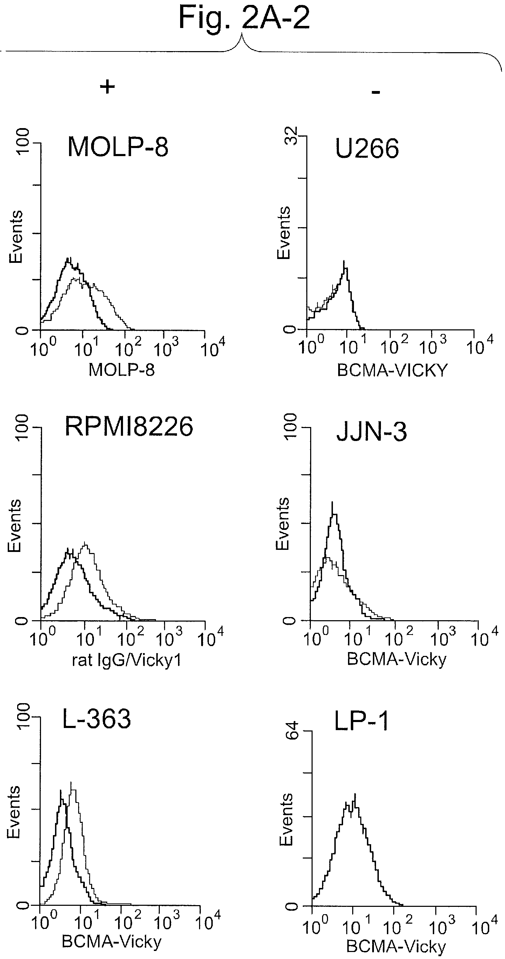

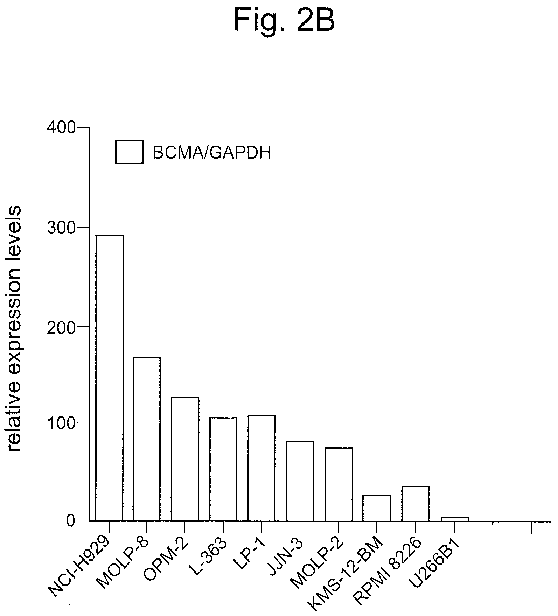

*Correlation of protein and mRNA expression levels in MM cell lines* As a next step, the inventors examined the level of BCMA protein as well as BCMA mRNA by FACS and human exon gene chip (Affymetrix), respectively in 10 different MM cell lines (Fig. 2).

mRNA expression levels were analyzed in RT-PCR using Primers described in Li et al (Med Oncol (2010) 27:439-445). In detail, total cellular RNA was isolated from MM cell as is commonly done in the art. The isolated RNA was applied as a template for the first strand cDNA synthesis by reverse transcription (RT), and cDNA used as a template for polymerase chain reaction (PCR). The following primers were used: BCMA 5'-TTA CTTGTCCTTCCAGGCTGTTCT-3' (sense) (SEQ ID NO:2) and 5'-CATAGAAACCAAGGAAGTTTCTACC-3' (antisense) (SEQ ID NO:3).

Fig. 2(A) shows the result of BCMA protein level by FACS using Vicky-1 in 10 MM cell lines. Intriguingly, BCMA protein level on the cell surface was varied in each cell line.

Especially, it was found that NCI-H929 (very strong indicated as +++ in Table 3), MOLP-2, OPM-2 as well as KMS-12-BM (strong indicated as ++ in Table 3) showed very strong or strong expression on the cell surface of said cell lines. On the other hand, U226B1, JJN-3 and LP-1 showed no BCMA protein expression on the cell surface of said cell lines.

Fig 2(B) indicates BCMA mRNA level of 10 different MM cell lines. The relative expression levels were resulted from the normalization by GAPDH mRNA level.
It was found that BCMA mRNA level was very high for example, in NCI-H929 cell line as it also showed high protein level. Although it was found that no expression of BCMA protein on the cell surface of U266B1, JJN-3 as well as LP-1 was detectable, BCMA mRNA in said cell lines were detectable, and it is noted that BCMA mRNA level of JJN-3 and LP-1 cell lines rather showed higher. Thus, the inventor concluded that there is no solid correlation between BCMA protein in the cell surface and BCMA mRNA in MM cell lines.

**Table 3: Summary of protein and mRNA expression levels in 10 different MM cell lines**

| | mRNA levels | Protein levels |
|---|---|---|
| NCI-H929 | +++ | +++ |
| MOLP-2 | ++ | ++ |
| OPM-2 | ++ | ++ |
| MOLP-8 | ++ | + |
| RPM18226 | + | + |
| KMS-12-BM | + | ++ |
| L-363 | ++ | + |
| U266B1 | + | - |
| JJ N-3 | ++ | - |
| LP-1 | ++ | - |

### BCMA protein expression on bone marrow plasma cells of MM patients

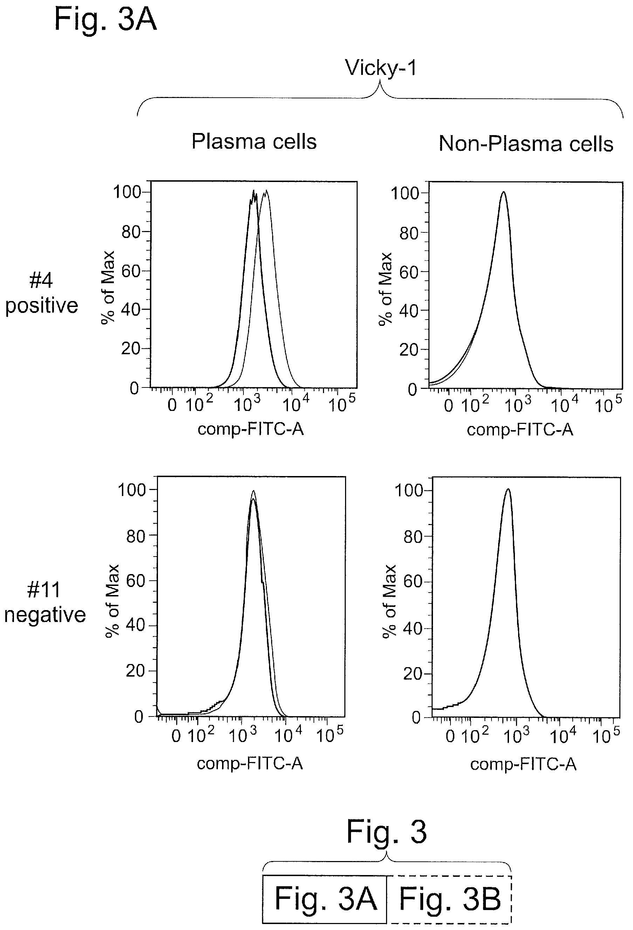

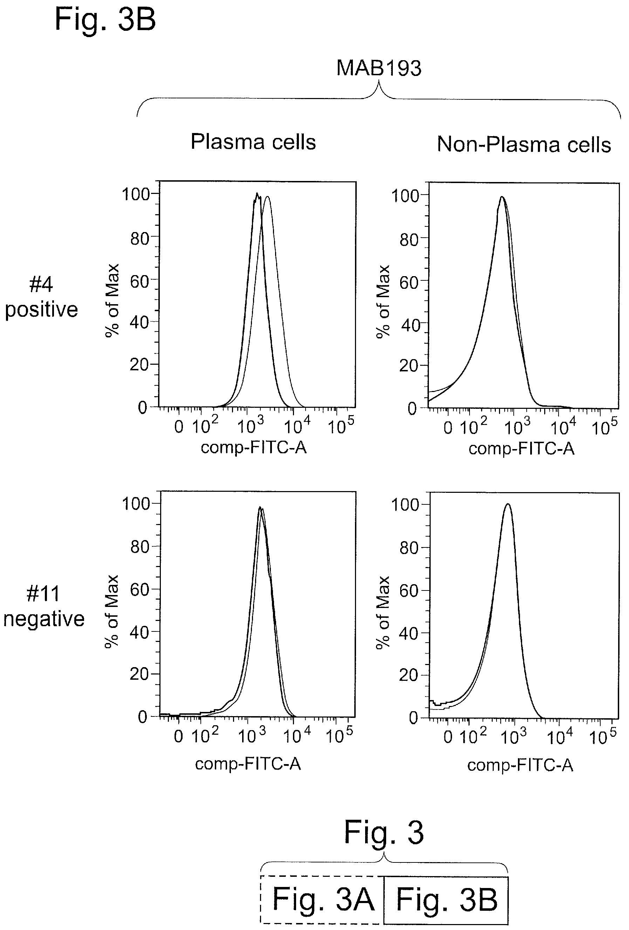

Further, the inventors examined as to whether or not BCMA protein was expressed on the cell surface of MM cells (malignant plasma B-cells) from 23 MM patients and detected by Vicky-1 or MAB193. Bone marrow aspirates of 23 patients were analyzed for BCMA expression in FACS. As a result, it was found that 52% (12/23) of MM patients showed BCMA positive on cell surface of MM cells, however, 48% (11/23) of MM patients showed BCMA negative (Table 4). In Fig. 3, the FACS signal shift was detected in primary plasma cells. On the other hand, no signal shift was observed in non-plasma cells (Fig. 3).

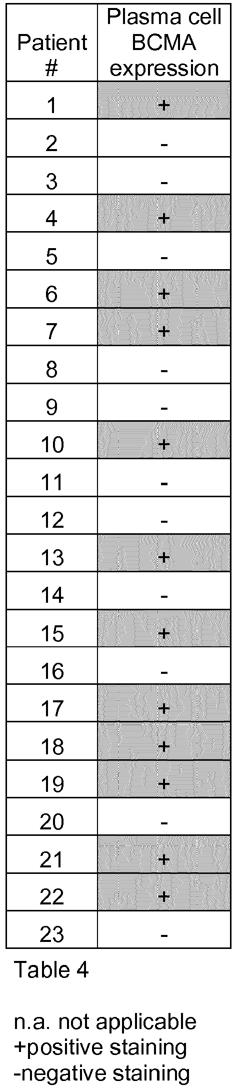

## Claims

1. A method for the stratification of a multiple myeloma (MM) patient disposed to respond favorably to an anti-BCMA antibody therapy, comprising determining whether B-cells, preferably malignant B-cells of said patient express BCMA protein on the surface of said B-cells, wherein said patient is disposed to respond favorably to said anti-BCMA antibody therapy, if said B-cells express BCMA protein on their surface.

2. A method for selecting an antibody-based multiple myeloma (MM) therapy, comprising determining whether B-cells, preferably malignant B-cells of a patient express BCMA protein on the surface of said B-cells, wherein, if said B-cells are BCMA positive, the patient may be subject to an anti-BCMA antibody therapy.

3. The method of claim 1 or claim 2, wherein said anti-BCMA antibody is
a monoclonal antibody,
a chimeric antibody
a humanized antibody, or
a bispecific antibody.

4. The method of claim 1 or claim 2, wherein said anti-BCMA antibody is conjugated to a toxin.

5. An anti-BCMA antibody for use in the treatment or amelioration of a multiple myeloma (MM) patient, wherein said patient has been determined to respond favorably to an anti-BCMA antibody therapy by carrying out the method of claim 1 or claim 2.

6. Use of a kit comprising an anti-BCMA antibody and instructions for carrying out the method of any one of claims 1 to 4.

## Patentansprüche

1. Verfahren zur Stratifizierung eines Multiplen Myelom (MM)-Patienten, der disponiert ist, positiv auf eine anti-BCMA-Antikörper-Therapie anzusprechen, umfassend die Ermittlung ob B-Zellen, vorzugsweise maligne B-Zellen des besagten Patienten BCMA-Protein auf der Oberfläche besagter B-Zellen exprimieren, wobei der Patient disponiert ist, positiv auf besagte anti-BCMA-Antikörper-Therapie anzusprechen, falls besagte B-Zellen BCMA-Protein auf ihrer Oberfläche exprimieren.

2. Verfahren zum Auswählen einer Antikörper-basierten Multiplen Myelom (MM)-Therapie, umfassend die Ermittlung ob B-Zellen, vorzugsweise maligne B-Zellen eines Patienten BCMA-Protein auf der Oberfläche besagter B-Zellen exprimieren, wobei der Patient, falls besagte B-Zellen BCMA-positiv sind, einer anti-BCMA-Antikörper-Therapie unterzogen werden kann.

3. Verfahren nach Anspruch 1 oder Anspruch 2, wobei besagter anti-BCMA-Antikörper
ein monoklonaler Antikörper,
ein chimärer Antikörper
ein humanisierter Antikörper, oder
ein bispezifischer Antikörper ist.

4. Verfahren nach Anspruch 1 oder Anspruch 2, wobei besagter anti-BCMA-Antikörper an ein Toxin konjugiert ist.

5. Anti-BCMA-Antikörper zur Verwendung in der Behandlung oder Besserung eines Multiplen Myelom (MM)-Patienten, wobei durch Ausführen des Verfahrens nach Anspruch 1 oder Anspruch 2 ermittelt wurde, dass besagter Patient positiv auf eine anti-BCMA-Antikörper-Therapie anspricht.

6. Verwendung eines Kits umfassend einen anti-BCMA-Antikörper und Anleitungen zum Ausführen des Verfahrens nach irgendeinem der Ansprüche 1 bis 4.

## Revendications

1. Méthode de stratification d'un patient souffrant de myélome multiple (MM) disposé à répondre favorablement à un traitement par un anticorps anti-BCMA, comprenant la détermination si les lymphocytes B, de préférence les lymphocytes B malins dudit patient expriment la protéine BCMA sur la surface desdits lymphocytes B, ledit patient étant disposé à répondre favorablement au dit traitement par un anticorps anti-BCMA, si lesdits lymphocytes B expriment la protéine BCMA sur leur surface.

2. Méthode de sélection d'un traitement à base d'anticorps de myélome multiple (MM), comprenant la détermination si les lymphocytes B, de préférence les lymphocytes B malins d'un patient expriment la protéine BCMA sur la surface desdits lymphocytes B, dans laquelle, si lesdits lymphocytes B sont positifs pour le BCMA, le patient peut être soumis à un traitement par un anticorps anti-BCMA.

3. Méthode selon la revendication 1 ou la revendication 2, dans laquelle ledit anticorps anti-BCMA est
un anticorps monoclonal,
un anticorps chimérique,
un anticorps humanisé, ou
un anticorps bispécifique.

4. Méthode selon la revendication 1 ou la revendication 2, dans laquelle ledit anticorps anti-BCMA est conjugué à une toxine.

5. Anticorps anti-BCMA pour son utilisation dans le traitement ou l'amélioration d'un patient souffrant de myélome multiple (MM), ledit patient ayant été déterminé comme répondant favorablement à un traitement par un anticorps anti-BCMA par réalisation de la méthode selon la revendication 1 ou la revendication 2.

6. Utilisation d'un kit comprenant un anticorps anti-BCMA et des instructions pour réaliser la méthode selon l'une quelconque des revendications 1 à 4.
